# EUROPEAN PATENT SPECIFICATION

(11) **EP 2 269 563 B1**
(45) Date of publication and mention of the grant of the patent: **27.04.2016**
(21) Application number: 10005158.0
(22) Date of filing: 18.05.2010
(51) Int. Cl.: A61J 15/00, A61M 39/02, A61M 39/10

(54) **Connecting structure for a gastrostomy catheter and a fluid supply tube**
Verbindungsstruktur für einen Gastrostomiekatheter und einen Flüssigkeitszuleitungsschlauch
Structure de connexion pour cathéter de gastrostomie et tube d'alimentation de fluide

(30) Priority: 29.06.2009 JP 2009153322
(43) Date of publication of application: 05.01.2011
(73) Proprietor: Covidien LP, Mansfield, MA 02048 (US)
(72) Inventor: Abe, Kazuhiro, Shizuoka 437-0004 (JP); Watanabe, Motonori, Shizuoka 437-0004 (JP)
(74) Representative: Tomlinson, Edward James

(56) References cited:
- EP-A2- 2 255 775
- WO-A1-2005/105017
- WO-A1-2008/003785

## Description

### [Technical Field]

The present invention relates to a connecting structure for a gastrostomy catheter and a fluid supply tube which are used for supplying a fluid such as nutrients or food in fluid form to the gastrointestinal tract of a patient.

### [Prior Art]

Fluids such as nutrients or food in fluid form are conventionally supplied to people having a reduced capacity for ingesting food orally by themselves due to advanced age or illness (referred to hereinafter as "patient(s)") using a gastrostomy catheter. The gastrostomy catheter generally comprises a tubular part which passes through a gastric fistula for ingestion which is formed in the patient's abdomen, an external holding part which is attached to the base end of the tubular part and placed on the surface of the skin at the abdomen in order to prevent the gastrostomy catheter from becoming embedded in the gastric fistula, and an internal holding part which is attached to the tip end of the tubular part and is placed inside the gastrointestinal tract, in the stomach for instance, in order to prevent the gastrostomy catheter from being removed from the gastric fistula. This kind of gastrostomy catheter is fitted in a gastric fistula formed in the patient's abdomen, and the wall of the gastrointestinal tract and the abdomen wall are fixed, after which the catheter is connected to a fluid supply tube. Fluid is then supplied to the gastrointestinal tract from this fluid supply tube via the gastrostomy catheter.

When the fluid supply tube is connected to this kind of gastrostomy catheter, if the fluid supply tube is pressed strongly into the gastrostomy catheter in order to make the tip end of the fluid supply tube fit together with the edge of the opening in the external holding part of the gastrostomy catheter, there is a risk of the external holding part of the gastrostomy catheter becoming embedded in the gastric fistula inside the body.

See for example WO2008/003785. In order to prevent the external fixing part from becoming embedded in the gastric fistula inside the body, the fluid supply tube needs to be connected to the gastrostomy catheter without the fluid supply tube strongly pressing the gastrostomy catheter. For this reason, there is a component in the gastrostomy catheter which is provided so that the fluid supply tube is made to engage with the external holding part of the gastrostomy catheter from a direction perpendicular to the longitudinal direction of the gastrostomy catheter (see Patent Document 1, for example).

With this catheter for establishing a gastric fistula (gastrostomy catheter), a flange is formed at the top of an adapter (external holding part), and legs which can engage with the flange of the catheter for establishing a gastric fistula are provided at the tip end of a feeding tube assembly (fluid supply tube). The feeding tube assembly is designed to be connected in a state in which it is perpendicular to the longitudinal direction of the catheter for establishing a gastric fistula, and the tip-end opening thereof is formed to be perpendicular to the feeding tube of the feeding tube assembly. A space which is the thickness of the flange from the tip-end opening is then provided in order to provide for the legs. Moreover, the base end of the legs is coupled to the tip end of the feeding tube assembly.

### [Prior Art Document]

### [Patent Document]

[Patent Document 1] Published Japanese Translation of a PCT Application 2008-504896

### [Summary of the Invention]

However, with the gastrostomy catheter described above, the fluid passage of the gastrostomy catheter and the fluid passage of the fluid supply tube which is connected to the gastrostomy catheter are at right-angles, and therefore the flow of fluid from the fluid supply tube to the gastrostomy catheter is not smooth. Furthermore, with conventional gastrostomy catheters, when people or objects touch the fluid supply tube which is connected to the gastrostomy catheter and the fluid supply tube is inadvertently pulled, the gastrostomy catheter may end up becoming removed from the patient's body if it is pulled together with the fluid supply tube.

The present invention, as claimed in claim 1, has been devised in order to resolve the problems mentioned above, and it aims to provide a connecting structure for a gastrostomy catheter and a fluid supply tube with which the gastrostomy catheter is not pressed into the body when a fluid supply tube is connected thereto and with which the gastrostomy catheter is not removed from the patient's body even if the fluid supply tube is pulled.

In order to achieve the aim described above, structural features of the connecting structure for a gastrostomy catheter and a fluid supply tube lie in the fact that it is a connecting structure for a gastrostomy catheter and a fluid supply tube, in which a fluid supply tube and a gastrostomy catheter which is fitted in a gastric fistula formed in the abdominal wall and the wall of the gastrointestinal tract of a patient are connected by way of a connecting member, the connecting structure for a gastrostomy catheter and a fluid supply tube being such that the section of the gastrostomy catheter which is disposed at the abdominal wall surface of the gastric fistula comprises an external holding part which has a part to be engaged formed at the outer periphery, and the fluid supply tube comprises a tube main body and a cylindrical part to be fixed which is provided on the outer periphery at the tip end of the tube main body; the connecting member comprises: an engaging part which can detachably engage with the part to be engaged and deforms by being pulled in the direction of extension of the gastrostomy catheter from a state of engagement with the part to be engaged so as to release the engagement with the part to be engaged; a guide part having a substantially C-shaped or U-shaped inner peripheral edge, in which the width between the two ends is set to be greater than the outer diameter of the tube main body and smaller than the outer diameter of the part to be fixed; and a linking part which links the guide part and the engaging part and is designed so that the space between the external holding part and the guide part is substantially the same as the length of the part to be fixed in the axial direction when the engaging part has been engaged with the part to be engaged; and when the tube main body is positioned within the guide part so that the part to be fixed is disposed between the external holding part and the guide part, the tip end of the fluid supply tube is placed in communication with an opening in the external holding part.

With the connecting structure for a gastrostomy catheter and a fluid supply tube according to the present invention, the part to be engaged is formed at the outer periphery of the external holding part of the gastrostomy catheter, and the cylindrical part to be fixed is provided at the tip end of the fluid supply tube which is connected to the gastrostomy catheter and supplies fluid to the gastrointestinal tract etc. of the patient by way of a fluid passage in the gastrostomy catheter. Furthermore, the connecting member for connecting the fluid supply tube to the external holding part of the gastrostomy catheter comprises a guide part of which the inner peripheral edge is substantially C-shaped or U-shaped, a deformable engaging part which detachably engages with the part to be engaged of the external holding part, and a linking part which links the guide part and the engaging part with a prescribed space between them.

Then, while the engaging part is engaged with the part to be engaged and the connecting member is assembled with the external holding part, the tip end section of the fluid supply tube is inserted into the guide part from the open side thereof so that the part to be fixed advances between the external holding part and the guide part, and this state is maintained, whereby the tip end of the fluid supply tube and the opening in the external holding part can be brought into a state of communication. In addition, the engagement between the engaging part and the part to be engaged can be released by pulling the fluid supply tube in this state in the direction of extension of the gastrostomy catheter to cause the engaging part to deform. Consequently, if the fluid supply tube is pulled with a specific force, the connecting member is removed from the external holding part together with the fluid supply tube.

Accordingly, the fluid supply tube can be connected to the gastrostomy catheter by moving the part to be fixed sideways in such a way that it slides between the external holding part and the guide part, and there is no need to press the fluid supply tube into the gastrostomy catheter. This means that when the fluid supply tube is connected to the gastrostomy catheter, the external holding part is not pressed into the gastric fistula inside the body, and does not become embedded therein. Furthermore, when the fluid supply tube has been connected to the gastrostomy catheter, the fluid passage of the gastrostomy catheter and the fluid passage of the fluid supply tube are linked in a substantially linear state. Consequently, there is no difficulty in the fluid flowing from the fluid supply tube to the gastrostomy catheter, and leaks are less likely to occur at the connection between the gastrostomy catheter and the fluid supply tube.

Furthermore, if the fluid supply tube is inadvertently pulled, the guide part of the connecting member is pulled by the part to be fixed, whereby the engagement between the engaging part and the part to be engaged is released. The gastrostomy catheter is therefore not pulled by the fluid supply tube, through the connecting member, and the gastrostomy catheter is not removed from the patient's body. That is to say, in the present invention, the engaging force between the engaging part and the part to be engaged is set to be smaller than the engaging force between the gastrostomy catheter and the gastric fistula. Furthermore, the guide part is preferably more rigid than the engaging part, which means that if the fluid supply tube is pulled, the force from the part to be fixed is reliably transmitted to the guide part. It should be noted that the deformation of the engaging part in accordance with the present invention includes extension/contraction and bending, and the deformable engaging part may be an extendible annular body, a plurality of hook-like bodies which are arranged spaced apart in a ring, or various kinds of engaging pieces which branch in two and in which the inner peripheral edge can engage with the part to be engaged.

Further structural features of the connecting structure for a gastrostomy catheter and a fluid supply tube lie in the fact that the part to be engaged comprises a groove or a projection which extends in a direction perpendicular to the direction of extension of the gastrostomy catheter, and the engaging part has a flexible section in which the inner peripheral edge is substantially C-shaped or U-shaped and in which a projection or a groove which can engage with the abovementioned groove or projection is formed on the inner peripheral edge; and the external holding part is designed to enter from the open side of the engaging part, and the engaging part is made to slidably engage with the part to be engaged in order to assemble the connecting member with the external holding part.

With the connecting structure for a gastrostomy catheter and a fluid supply tube according to the present invention, the part to be engaged, which comprises a groove or a projection that extends in a direction perpendicular to the direction of extension of the gastrostomy catheter, i.e. in a direction perpendicular to the fluid passage provided in the gastrostomy catheter, is formed at the outer periphery of the external holding part of the gastrostomy catheter, and the engaging part of the connecting member has a flexible section in which the inner peripheral edge, which can slidably engage with the part to be engaged of the external holding part, is substantially C-shaped or U-shaped. Consequently, the external holding part is inserted into the engaging part from the open side thereof so that the part to be engaged engages with the engaging part, whereby the connecting member can be attached to the external holding part.

Accordingly, the connecting member can be attached to the external holding part of the gastrostomy catheter by moving the connecting member sideways in such a way that it slides with respect to the external holding part, and there is no need to press the gastrostomy catheter into the body. This means that when the connecting member is attached to the gastrostomy catheter, the external holding part is not pressed into the gastric fistula inside the body, and does not become embedded therein. Furthermore, if the fluid supply tube is inadvertently pulled, the guide part of the connecting member is pulled by the part to be fixed, and the engaging part bends so that the engagement between the engaging part and the part to be engaged is released. Consequently, the gastrostomy catheter is not removed from the patient's body if it is pulled by the fluid supply tube.

Further structural features of the connecting structure for a gastrostomy catheter and a fluid supply tube lie in the fact that the gastrostomy catheter is provided with the external holding part, a tubular part of which the base end is joined to the external holding part and the tip end section is disposed so as to extend from the gastric fistula into the gastrointestinal tract, and an internal holding part which is joined to the tubular part and is disposed within the gastrointestinal tract. This means that the internal holding part prevents the gastrostomy catheter from being removed from the gastric fistula, and therefore the gastrostomy catheter is even less likely to be removed from the gastric fistula in the patient.

### [Brief Description of the Figures]

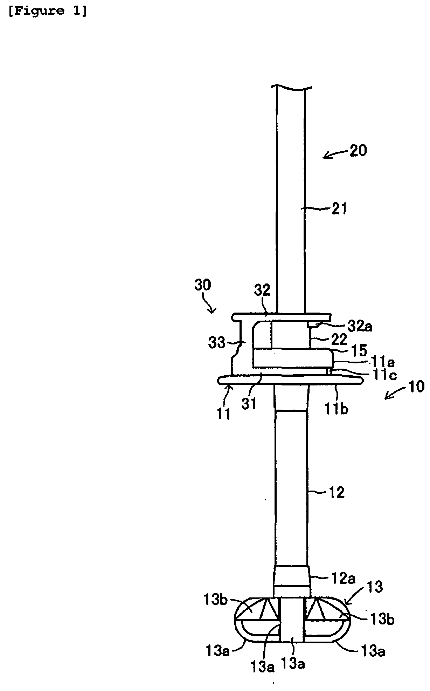

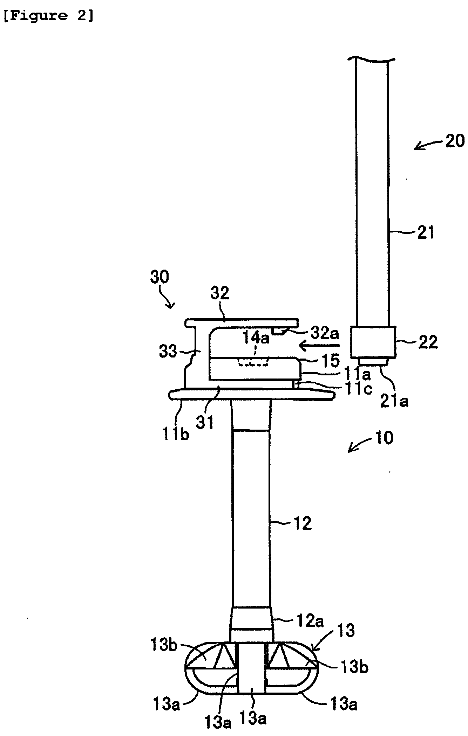

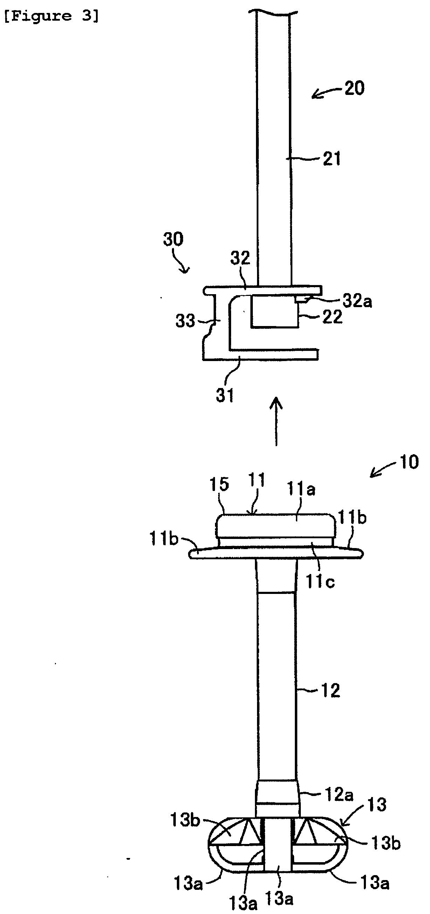

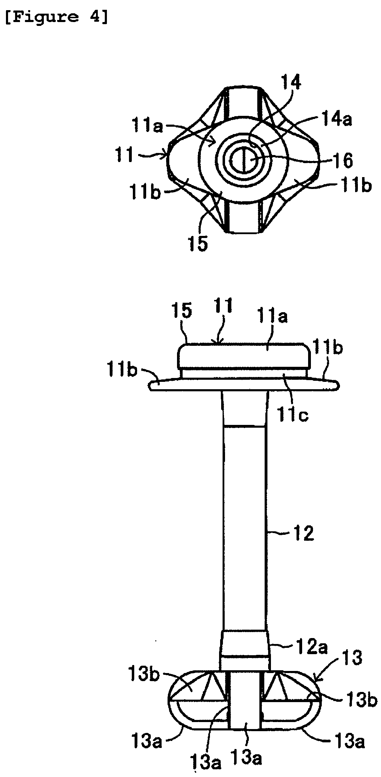

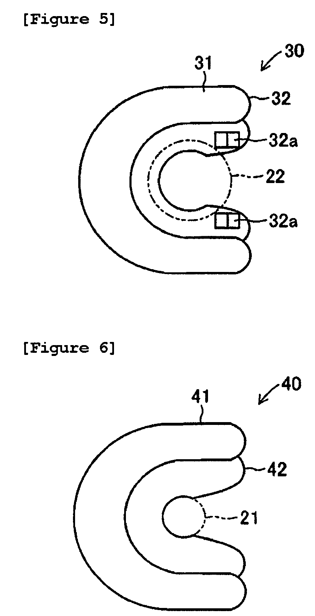

[Figure 1] is a front view showing a gastrostomy catheter and a fluid supply tube when they are connected by the connecting structure for a gastrostomy catheter and a fluid supply tube according to a first mode of embodiment of the present invention; [Figure 2] is a front view showing the state before the gastrostomy catheter and fluid supply tube shown in Figure 1 are connected; [Figure 3] is a front view showing a state in which the connecting member has been removed from the gastrostomy catheter together with the fluid supply tube; [Figure 4] shows the gastrostomy catheter, where (a) is a plan view and (b) is a front view; [Figure 5] is a bottom view of the connecting member; and [Figure 6] is a bottom view showing the connecting member according to a second mode of embodiment.

### [Modes of Embodiment of the Invention]

### (First Mode of Embodiment)

A first mode of embodiment of the present invention will be described below with the aid of the figures. Figures 1 to 3 show a gastrostomy catheter 10, fluid supply tube 20 and connecting member 30 which make up the connecting structure for a gastrostomy catheter and a fluid supply tube, in accordance with this mode of embodiment. As shown in Figure 4, the gastrostomy catheter 10 comprises an external holding part 11, a tubular part 12 which is joined at the centre on the lower surface of the external holding part 11, and an internal holding part 13 which is joined to the tip end of the tubular part 12, all of these components being made of polyurethane. In the description that follows, the external holding part 11 side shall be referred to as the upper side/top/above, and the internal holding part 13 side shall be referred to as the lower side/bottom/below.

The external holding part 11 comprises an insertion opening 11a which is formed as a fairly thick annular shape, and a pair of protrusions 11b which project outwards from both sides at the lower end on the outer periphery of the insertion opening 11a. The protrusions 11b have the function of preventing the gastrostomy catheter 10 from becoming embedded inside the patient's body. Furthermore, a part to be engaged 11c for the attachment of the connecting member 30 is formed in the peripheral direction on the upper side of the protrusions 11b, at the outer peripheral surface of the insertion opening 11a. This part to be engaged 11c has a groove-like shape, and the section of the insertion opening 11a where the part to be engaged 11c is formed has a smaller outer diameter than the section above the part to be engaged 11c.

An insertion hole 14 running vertically is then formed at the centre of the insertion opening 11a, and the diameter of an upper opening 14a of the insertion hole 14 becomes steadily greater from the bottom towards the top. Furthermore, the diameter of the top part on the outer periphery of the insertion opening 11a becomes steadily smaller from the bottom towards the top, and by means of this the centre of the top part on the outer periphery of the insertion opening 11a is formed as a raised curved face. By forming the tapered upper opening 14a and the curved face at the top part on the outer periphery, an annular raised part 15 is formed at the upper face of the insertion opening 11a. A check valve 16 which has a slit formed in the centre is then provided on the inner peripheral surface of the insertion hole 14.

The tubular part 12 consists of an elongate cylindrical body inside which is formed a fluid passage (not depicted) for the passage of fluids such as nutrients and food in fluid form, and the upper end of the fluid passage communicates with the insertion hole 14 of the external holding part 11. The internal holding part 13 is connected to the tubular part 12 by way of a cylindrical connector 12a which is fixed to the lower end of the tubular part 12. The internal holding part 13 comprises four strip-shaped linking parts 13a which extend in four directions from the edge of the opening at the lower end of the connector 12a, and four linking film parts 13b which are provided between the upper parts of each of the linking parts 13a, and the tip ends of the linking parts 13a are linked to one another.

The fluid supply tube 20 comprises a tube main body 21 and a part to be fixed 22 which is attached at the outer periphery towards the lower end of the tube main body 21. The tube main body 21 consists of a soft tube having a fluid passage which communicates with the fluid passage of the tubular part 12 by way of the insertion hole 14 of the external holding part, and it is substantially the same thickness as the tubular part 12. Furthermore, the tip end 21a of the tubular main body 21 enters the part to be fixed 22 by contracting under pressure in the axial direction.

In addition, the tip end 21a of the tube main body 21 can be fitted in a liquid-tight state with the upper section of the external holding part 11 including the upper opening 14a of the insertion hole 14, and this allows fluid to flow from the fluid passage of the tube main body 21 to the fluid passage of the gastrostomy catheter 10. Moreover, the slit of the check valve 16 is opened by the flow of fluid from the tube main body 21 to the gastrostomy catheter 10. The part to be fixed 22 consists of a cylindrical body made of a rigid resin material, and it is fixed to the outer periphery at the lower end of the tube main body 21 with the tip end 21a of the tube main body 21 able to be received therein.

The connecting member 30 comprises an engaging part 31 which can slidably engage with the part 11c to be engaged, a guide part 32 which is disposed parallel to and spaced apart from the engaging part 31, and a linking part 33 which links the engaging part 31 and the guide part 32. Figure 5 shows the connecting member 30 seen from the bottom, and the engaging part 31 consists of a flexible and elastic substantially U-shaped plate. The inner peripheral edge of the engaging part 31 then enters the part 11c to be engaged and is made to slide so that the external holding part 11 enters the engaging part 31 from the substantially U-shaped open side thereof (the right-hand side in Figure 5), and the section of the engaging part 31 within the inner peripheral edge is fitted into the part 11c to be engaged, whereby the connecting member 30 is rotatably assembled with the external holding part 11 of the gastrostomy catheter 10. Note that the inner peripheral edge of the engaging part 31 constitutes the projection according to the present invention.

Furthermore, when the connecting member 30 is pulled upwards with a specific force while the connecting member 30 is assembled with the external holding part 11, the sections at the two ends of the engaging part 31 bend, and the engagement between the part 11c to be engaged of the external holding part 11 and the engaging part 31 is released, as shown in Figure 3. It should be noted that when the connecting member 30 is removed from the external holding part 11 under normal circumstances, the connecting member 30 is made to slide in the opposite direction to when it is attached. The guide part 32 comprises a flexible and elastic plate which is substantially C-shaped when seen from above (not depicted), and it is disposed parallel to the engaging part 31 and spaced apart from the engaging part 31. The guide part 32 is somewhat more rigid than the engaging part 31.

The space between the engaging part 31 and the guide part 32 is set so that the space between the upper surface of the raised part 15 of the external holding part 11 and the lower surface of the guide part 32 is substantially equal to the length of the part to be fixed 22 in the axial direction when the engaging part 31 has been fitted into the part to be engaged 11c. Furthermore, the inner peripheral edge of the guide part 32 is formed in such a way that that the width between opposing sections on the open side (the right-hand section in Figure 5) becomes steadily narrower moving from the open side to the inside, and the inside thereof is formed to become substantially circular (C-shaped). Respective protrusions 32a are then formed at both ends on the lower surface of the guide part 32.

When the lower surfaces of these protrusions 32a are seen from the side, the portions lying on the open side of the guide part 32 are formed as tapers which are oriented steadily downwards moving from the open side to the inside of the guide part 32, and the portions lying on the inside of the guide part 32 are formed to be horizontal. Furthermore, the spacing of the pair of protrusions 32a is somewhat narrower than the outer diameter of the part to be fixed 22. As shown by the two-dot chain line in Figure 5, when the part to be fixed 22 has engaged with the guide part 32, the pair of protrusions 32a are in contact with the outer peripheral surface of the part to be fixed 22, and the part to be fixed 22 is prevented from being removed from the guide part 32. The linking part 33 links the central part of the top face of the engaging part 31 and the central part of the bottom face of the guide part 32, and it consists of a platelike linking piece which has an arcuate shape in cross section (not depicted). Furthermore, the width between the two ends of the guide part 32 is set to be greater than the outer diameter of the tube main body 21 and smaller than the outer diameter of the part to be fixed 22.

With this configuration, when the fluid supply tube 20 is connected to the gastrostomy catheter 10 which has been fitted to the gastric fistula (not depicted) in the patient, the engaging part 31 is first of all fitted into the part to be engaged 11c of the external fitting part 11 to assemble the connecting member 30 with the gastrostomy catheter 10. In this case, the two ends on the open side of the engaging part 31 are pushed into the two sides of the part to be engaged 11c, and the connecting member 30 is made to slide sideways with respect to the gastrostomy catheter 10. By means of this, the inside section on the inner peripheral edge of the engaging part 31 fits into the semicircular portion of the part to be engaged 11c, and the connecting member 30 is assembled in a state in which it can rotate in the axial direction with respect to the gastrostomy catheter 10. When this assembly takes place, the gastrostomy catheter 10 is not pressed into the patient's body, and therefore the external holding part 11 does not become embedded in the gastric fistula.

Next, as shown in Figure 2, the part to be fixed 22 is positioned in the region between the open side section of the guide part 32 of the connecting member 30 and the raised part 15 of the gastrostomy catheter 10, and the fluid supply tube 20 is brought closer to the gastrostomy catheter 10. Then, as shown by the arrow, the fluid supply tube 20 is moved towards the gastrostomy catheter 10, and the part to be fixed 22 is pushed between the raised part 15 and the guide part 32. At this point, the tip end 21a of the tube main body 21 is pressed by the raised part 15 and is received inside the part to be fixed 22, and the part to be fixed 22 enters between the raised part 15 and the guide part 32 while the upper ends on both sides of the part to be fixed 22 (the two sides at the front and rear in Figure 2) are pushed up against the tapered faces of the protrusions 32a.

At this point, the open side portion of the guide part 32 is bent upwards. Moreover, the force of the two sides at the upper end of the part to be fixed 22 pushing the tapered surfaces of the protrusions 32a upwards at this time is preferably less than the force allowing the release of the engagement between the engaging part 31 and the part to be engaged 11c. Even if the force of the part to be fixed 22 pushing the protrusions 32a upwards is greater than the force allowing the release of the engagement between the engaging part 31 and the part to be engaged 11c, the connecting member 30 can be supported with the hand in order to prevent the release of the engagement between the engaging part 31 and the part to be engaged 11c. Then, when the two sides at the upper end of the part to be fixed 22 have gone past the protrusions 32a, the tip end 21a of the tube main body 21 protrudes from the part to be fixed 22, and fits together with the upper section of the insertion hole 14 of the external holding part 11, and the state shown in Figure 1 is reached.

At this point, the tip end 21a of the tube main body 21 is in a state of liquid-tight contact with the upper opening 14a. Furthermore, the guide part 32 returns to its original state, and the protrusions 32a engage with the outer peripheral surface of the part to be fixed 22, as shown in Figure 5. This means that the part to be fixed 22 is prevented from being removed from the gastrostomy catheter 10. When the fluid supply tube 20 is connected to the gastrostomy catheter 10, the gastrostomy catheter 10 is not pushed into the patient's body, and therefore the external holding part 11 does not become embedded in the gastric fistula.

When the fluid supply tube 20 and the gastrostomy catheter 10 are in a connected state, fluid enters the tube main body 21 from the base end opening of the tube main body 21. Note that a supply apparatus housing the fluid has already been connected to the base end opening of the tube main body 21, and the fluid is supplied from the supply apparatus. As a result, the fluid is supplied from the fluid passage of the tube main body 21, through the insertion hole 14 and fluid passage of the tubular part 12, and into the patient's gastrointestinal tract, for example into the stomach. Furthermore, if fluid or soiling adheres to the external holding part 11 of the gastrostomy catheter 10, the fluid or soiling is wiped off using a specific cloth or paper. In this case, the connecting member 30 is turned to allow the whole surface of the external holding member 11 to be wiped.

Furthermore, when the fluid supply tube is connected to the gastrostomy catheter 10, if an object touches the fluid supply tube 20 or the fluid supply tube 20 is inadvertently pulled as the patient changes position, the sections at the two sides of the engaging part 31 bend so that the engagement between the part to be engaged 11c of the external holding part 11 and the engaging part 31 is released. As a result, the connecting member 30 is detached form the external holding part 11 together with the fluid supply tube 20, as shown in Figure 3. This means that the gastrostomy catheter 10 is prevented from being removed from the gastric fistula if the external holding part 11 is pulled outside the patient's body.

As described above, with the connecting structure for a gastrostomy catheter and a fluid supply tube according to the present invention, the connecting member 30 can be assembled with the gastrostomy catheter 10 by inserting both ends on the open side of the engaging part 31 into the two side sections of the part to be engaged 11c and sliding the connecting member 30 sideways with respect to the gastrostomy catheter 10. Then, when the connecting member 30 is in an assembled state with the gastrostomy catheter 10, the tip end section of the fluid supply tube 20 is inserted into the guide part 32 from the open side of the guide tube 32, and the part to be fixed 22 is pushed between the external holding part 11 and the guide part 32, and this state is maintained, whereby the tip end 21a of the fluid supply tube can be brought into communication with the upper opening 14a of the external holding part 11.

Accordingly, the connecting member 30 can be assembled with the gastrostomy catheter 10 by making the connecting member 30 slide sideways with respect to the gastrostomy catheter 10, and the fluid supply tube 20 can be connected to the gastrostomy catheter 10 by moving the part to be fixed 22 sideways in such a way that it slides between the external holding part 11 and the guide part 32, and there is no need to press the fluid supply tube 20 into the gastrostomy catheter 10. This means that when the fluid supply tube 20 is connected to the gastrostomy catheter 10, the external holding part 11 is not pressed into the gastric fistula inside the body, and does not become embedded therein.

Furthermore, when the fluid supply tube 20 has been connected to the gastrostomy catheter 10, the fluid passage of the gastrostomy catheter 10 and the fluid passage of the fluid supply tube 20 are linked in a substantially linear state. Consequently, there is no difficulty in the fluid flowing from the fluid supply tube 20 to the gastrostomy catheter 10, and leaks are less likely to occur at the connection between the gastrostomy catheter 10 and the fluid supply tube 20. This means that fluids can be smoothly supplied into the gastrointestinal tract of a patient.

In addition, if the fluid supply tube 20 is inadvertently pulled, the guide part 32 of the connecting member 30 is pulled by the part to be fixed 22, whereby the engagement between the engaging part 31 and the part to be engaged 11c is released. Consequently, the connecting member 30 is detached from the external holding part 11 together with the fluid supply tube 22. As a result, the gastrostomy catheter 10 is not removed from the patient's body when the catheter is pulled by the fluid supply tube 20. The gastrostomy catheter 10 further comprises the internal holding part 13, and therefore the gastrostomy catheter 10 is even less likely to be removed from the patient's body.

### (Second Mode of Embodiment)

Figure 6 shows a connecting member 40 of a connecting structure for a gastrostomy catheter and a fluid supply tube according to a second mode of embodiment of the present invention. This connecting member 40 comprises an engaging part 41 which can slidably engage with the part 11c to be engaged, a guide part 42 which is disposed parallel to and spaced apart from the engaging part 41, and a linking part (not depicted) which links the engaging part 41 and the guide part 42. The engaging part 41 comprises the same substantially U-shaped plate as the engaging part 31 described above. The guide part 42 comprises a substantially C-shaped plate when seen from above (not depicted), and it is disposed parallel to the engaging part 41 and spaced apart from the engaging part 41.

Furthermore, in the same way as for the inner peripheral edge of the guide part 32 described above, the inner peripheral edge of the guide part 42 is formed in such a way that that the width between opposing sections on the open side (the right-hand section in Figure 5) becomes steadily narrower moving from the open side to the inside, and the inside thereof is formed to become substantially circular (C-shaped), but the circular section on the inside is smaller than the circular section of the guide part 32. When the part to be fixed 22 has been assembled with the connecting member 40, the tube main body 21 is pushed in and engages with the circular section on the inside of the inner peripheral edge of the guide part 42, as shown by the two-dot chain line in Figure 6, and the fluid supply tube 20 is prevented from becoming removed from the gastrostomy catheter 10 and the connecting member 40. Note that this guide part 42 is not provided with protrusions.

The structures of the other components of the connecting structure for a gastrostomy catheter and a fluid supply tube according to this mode of embodiment are the same as those of the connecting structure for a gastrostomy catheter and a fluid supply tube according to the first mode of embodiment described above. Furthermore, with the connecting structure for a gastrostomy catheter and a fluid supply tube according to this mode of embodiment, the part to be fixed 22 engages between the external holding part 11 and the guide part 32, and the tube main body 21 engages within the inner peripheral edge of the guide part 42. The other operational effects of the connecting structure for a gastrostomy catheter and a fluid supply tube according to this mode of embodiment are the same as those of the connecting structure for a gastrostomy catheter and a fluid supply tube according to the first mode of embodiment described above.

The connecting structure for a gastrostomy catheter and a fluid supply tube according to the present invention is not limited to the modes of embodiment described above, and suitable modifications may be made within the technical scope of the present invention. For example, in the modes of embodiment described above, the connecting member 30 is able to rotate in the axial direction with respect to the gastrostomy catheter 10, but the part to be engaged 11c may consist of two grooves which are parallel rather than circular, and the connecting member 30 may not be able to rotate. Furthermore, the part to be engaged 11c need not be a groove, and it may be a projection, and a groove which can engage with the part to be engaged 11c, which is a projection, may be formed at the inner peripheral edge of the engaging parts 31, 41.

In addition, instead of the engaging parts 31, 41, use may be made of extendible annular members, or a plurality of rod-like linking parts may extend downwards from the guide parts 32, 42 and hook-like parts may be provided at the lower end thereof. In addition, a cap which can fit onto the upper opening 14a may be provided on the external holding part 11 of the gastrostomy catheter 10 so that the upper opening 14 can be closed off by the cap when the fluid supply tube 20 is not connected to the gastrostomy catheter 10.

### [Key to Symbols]

10... gastrostomy catheter; 11...external holding part; 11c...part to be engaged; 12...tubular part; 13...internal holding part; 14a...upper opening; 20...fluid supply tube; 21...tube main body; 21a...tip end; 22...part to be fixed; 30, 40...connecting member; 31, 41...engaging part; 32, 42...guide part.

## Claims

1. Connecting structure for a gastrostomy catheter (10) and a fluid supply tube (20), in which a fluid supply tube (20) and a gastrostomy catheter (10) which is fitted in a gastric fistula formed in the abdominal wall and the wall of the gastrointestinal tract of a patient are connected by way of a connecting member (30),
the connecting structure for a gastrostomy catheter (10) and a fluid supply tube (20) a being **characterized in that** the section of the gastrostomy catheter which is disposed at the abdominal wall surface of the gastric fistula comprises an external holding part (11) which has a part to be engaged (11c) formed at the outer periphery, and the fluid supply tube (20) comprises a tube main body (21) and a cylindrical part to be fixed (22) which is provided on the outer periphery at the tip end of the tube main body (21)
the connecting member (30) comprises: an engaging part (31) which can detachably engage with the part to be engaged (11c) and deforms by being pulled in the direction of extension of the gastrostomy catheter (10) from a state of engagement with the part to be engaged (11c) so as to release the engagement with the part to be engaged (11c); a guide part (32) having a substantially C-shaped or U-shaped inner peripheral edge, in which the width between the two ends is set to be greater than the outer diameter of the tube main body (21) and smaller than the outer diameter of the part to be fixed (22); and a linking part (33) which links the guide part (32) and the engaging part (31) and is designed so that the space between the external holding part (11) and the guide part (32) is substantially the same as the length of the part to be fixed (22) in the axial direction when the engaging part (31) has been engaged with the part to be engaged(11c); and when the tube main body (21) is positioned within the guide part (32) so that the part to be fixed (22) is disposed between the external holding part (11) and the guide part (32) , the tip end (21a) of the fluid supply tube (20) is placed in communication with an opening in the external holding part (11).

2. Connecting structure for a gastrostomy catheter and a fluid supply tube, according to Claim 1, in which the part to be engaged (11c) comprises a groove or a projection which extends in a direction perpendicular to the direction of extension of the gastrostomy catheter (10), and the engaging part (31) has a flexible section in which the inner peripheral edge is substantially C-shaped or U-shaped and in which a projection or a groove which can engage with the abovementioned groove or projection is formed on the inner peripheral edge; and the external holding part (11) is designed to enter from the open side of the engaging part (31), and the engaging part (31) is made to slidably engage with the part to be engaged (11c) in order to assemble the connecting member (30) with the external holding part (11).

3. Connecting structure for a gastrostomy catheter and a fluid supply tube, according to Claim 1 or 2, in which the gastrostomy catheter (10) is provided with the external holding part (11), a tubular part (12) of which the base end is joined to the external holding part (11) and the tip end section is disposed so as to extend from the gastric fistula into the gastrointestinal tract, and an internal holding part (13) which is joined to the tubular part (12) and is disposed within the gastrointestinal tract.

## Patentansprüche

1. Verbindungsstruktur für einen Gastrostomiekatheter (10) und einen Flüssigkeitszufuhrschlauch (20), in welchem ein Flüssigkeitszufuhrschlauch (20) und ein Gastrostomiekatheter (10), der in einer in der Bauchwand gebildeten Magenfistel und der Wand des Magen-Darm-Trakts eines Patienten eingepasst ist, durch ein Verbindungselement (30) miteinander verbunden sind, wobei die Verbindungsstruktur für einen Gastrostomiekatheter (10) und einen Flüssigkeitszufuhrschlauch (20) **dadurch gekennzeichnet ist, dass** der Abschnitt des Gastrostomiekatheters, der an der Bauchwandoberfläche der Magenfistel angeordnet ist, ein externes Halteteil (11) umfasst, das ein einzugreifendes Teil (11c) aufweist, das am äußeren Umfang gebildet ist, und wobei der Flüssigkeitszufuhrschlauch (20) einen Schlauchhauptkörper (21) sowie ein zu fixierendes zylindrisches Teil (22) umfasst, das am äußeren Umfang am Spitzenende des Schlauchhauptkörpers (21) bereitgestellt ist,
wobei das Verbindungselement (30) Folgendes umfasst: ein eingreifendes Teil (31), das lösbar in das einzugreifende Teil (11c) eingreifen kann und sich verformt, indem es in die Richtung der Erstreckung des Gastrostomiekatheters (10) aus einem Zustand des Eingreifens mit dem einzugreifenden Teil (11c) derart gezogen wird, um den Eingriff in das einzugreifende Teil (11c) zu lösen; ein Führungsteil (32) mit einer im Wesentlichen C-förmigen oder U-förmigen inneren peripheren Kante, in welcher die Breite zwischen den beiden Enden größer eingestellt wird, als der Außendurchmesser des Schlauchhauptkörpers (21) ist und kleiner, als der Außendurchmesser des zu fixierenden Teils (22) ist; und ein Kopplungsteil (33), welches das Führungsteil (32) und das eingreifende Teil (31) miteinander koppelt und derart ausgelegt ist, sodass der Raum zwischen dem externen Halteteil (11) und dem Führungsteil (32) im Wesentlichen derselbe ist, wie die Länge des zu fixierenden Teils (22) in der axialen Richtung, wenn das eingreifende Teil (31) in das einzugreifende Teil (11c) eingegriffen hat; und wenn der Schlauchhauptkörper (21) innerhalb des Führungsteils (32) derart positioniert ist, sodass das zu fixierende Teil (22) zwischen dem externen Halteteil (11) und dem Führungsteil (32) angeordnet ist, wobei das Spitzenende (21a) des Flüssigkeitszufuhrschlauchs (20) in Kommunikation mit einer Öffnung im externen Halteteil (11) versetzt wird.

2. Verbindungsstruktur für einen Gastrostomiekatheter und einen Flüssigkeitszufuhrschlauch nach Anspruch 1, wobei das einzugreifende Teil (11c) eine Rille oder einen Vorsprung umfasst, die/der'sich in einer Richtung senkrecht zur Richtung der Erstreckung des Gastrostomiekatheters (10) erstreckt und wobei das eingreifende Teil (31) einen flexiblen Abschnitt aufweist, in dem die innere periphere Kante im Wesentlichen C-förmig oder U-förmig ist und wobei ein Vorsprung oder eine Rille, der/die in die oben genannte Rille oder den Vorsprung eingreifen kann, an der inneren peripheren Kante gebildet ist; und wobei das externe Halteteil (11) derart ausgelegt ist, um von der offenen Seite des eingreifenden Teils (31) aus einzutreten und das eingreifende Teil (31) derart hergestellt ist, um gleitend in das einzugreifende Teil (11c) einzugreifen, um das Verbindungselement (30) mit dem externen Halteteil (11) zusammenzufügen.

3. Verbindungsstruktur für einen Gastrostomiekatheter und einen Flüssigkeitszufuhrschlauch nach Anspruch 1 oder 2, wobei der Gastrostomiekatheter (10) mit dem externen Halteteil (11), einem schlauchartigen Teil (12), dessen Sockelende mit dem externen Halteteil (11) verbunden ist und der Spitzenendabschnitt derart angeordnet ist, um sich von der Magenfistel in den Magen-Darm-Trakt zu erstrecken, sowie einem internen Halteteil (13), das mit dem schlauchartigen Teil (12) verbunden und innerhalb des Magen-Darm-Trakts angeordnet ist, bereitgestellt ist.

## Revendications

1. Structure de connexion pour un cathéter de gastrotomie (10) et un tube d'alimentation en fluide (20) dans laquelle un tube d'alimentation en fluide (20) et un cathéter de gastrostomie (10) qui est monté dans une fistule gastrique formée dans la paroi abdominale et la paroi du tractus gastro-intestinal d'un patient sont raccordés par l'intermédiaire d'un élément de connexion (30),
la structure de connexion pour un cathéter de gastrotomie (10) et un tube d'alimentation en fluide (20) étant **caractérisée en ce que** la section du cathéter de gastrostomie qui est disposé à la surface de la paroi abdominale de la fistule gastrique comprend une pièce de fixation externe (11) qui présente une partie destinée à être mise en prise (11c) formée à la périphérie extérieure, et le tube d'alimentation en fluide (20) comprend un corps principal de tube (21) et une partie cylindrique destinée à être fixée (22) qui est prévue sur la périphérie extérieure à l'extrémité de l'embout du corps principal de tube (21),
l'élément de connexion (30) comprend : une partie de mise en prise (31) qui peut venir en prise de manière amovible avec la partie destinée à être mise en prise (11c) et se déforme en étant tirée dans la direction d'extension du cathéter de gastrotomie (10) à partir d'un état de mise en prise avec la partie destinée à être mise en prise (11c) de façon à libérer la mise en prise avec la partie destinée à être mise en prise (11c) ;
une pièce de guidage (32) ayant un bord périphérique intérieur sensiblement en forme de C ou en forme de U, dans laquelle la largeur entre les deux extrémités est définie pour être supérieure au diamètre extérieur du corps principal de tube (21) et inférieure au diamètre extérieur de la partie destinée à être fixée (22) ;
et une partie de liaison (33) qui relie la pièce de guidage (32) et la partie de mise en prise (31) et est conçue de telle sorte que l'espace entre la pièce de fixation externe (11) et la pièce de guidage (32) soit sensiblement identique à la longueur de la partie destinée à être fixée (22) dans la direction axiale lorsque la partie de mise en prise (31) a été mise en prise avec la partie destinée à être mise en prise (11c) ;
et lorsque le corps principal de tube (21) est positionné à l'intérieur de la pièce de guidage (32) de sorte que la partie destinée à être fixée (22) soit disposée entre la pièce de fixation externe (11) et la pièce de guidage (32), l'extrémité de l'embout (21a) du tube d'alimentation en fluide (20) est placée en communication avec une ouverture dans la pièce de fixation externe (11).

2. Structure de connexion pour un cathéter de gastrotomie et un tube d'alimentation en fluide, selon la revendication 1, dans laquelle la partie destinée à être mise en prise (11c) comprend une rainure ou une saillie qui se prolonge dans une direction perpendiculaire à la direction d'extension du cathéter de gastrotomie (10), et la partie de mise en prise (31) présente une section flexible, dans laquelle le bord périphérique intérieur est sensiblement en forme de C ou en forme de U et dans laquelle une saillie ou une rainure qui peut venir en prise avec la rainure ou la saillie précitée est formée sur le bord périphérique intérieur ;
et la pièce de fixation externe (11) est destinée à entrer dans le côté ouvert de la partie de mise en prise (31), et la partie de mise en prise (31) est destinée à venir en prise de manière coulissante avec la partie destinée à être mise en prise (11c) afin d'assembler l'élément de connexion (30) avec la pièce de fixation externe (11).

3. Structure de connexion pour un cathéter de gastrotomie et un tube d'alimentation en fluide, selon la revendication 1 ou 2, dans laquelle le cathéter de gastrotomie (10) est équipé de la pièce de fixation externe (11), d'une partie tubulaire (12) dont l'extrémité de base est fixée à la pièce de fixation externe (11) et la section d'extrémité de l'embout est disposée de manière à s'étendre à partir de la fistule gastrique dans le tractus gastro-intestinal, et d'une pièce de fixation interne (13) qui est fixée à la partie tubulaire (12) et est disposée dans le tractus gastro-intestinal.
